# EUROPÄISCHE PATENTANMELDUNG

(11) **EP 2 682 157 A1**
(43) Veröffentlichungstag der Anmeldung: **08.01.2014**
(21) Anmeldenummer: 12005045.5
(22) Anmeldetag: 06.07.2012
(51) Int. Cl.: A61N 1/05, A61B 18/14

(54) **Mapping Elektrode für den linken Vorhof des Herzens**

(71) Anmelder: Peter Osypka Stiftung, 79639 Grenzach-Wyhlen (DE)
(72) Erfinder: Osypka, Peter, Dr., 79639 Grenzach-Wyhlen (DE)

(57) **Zusammenfassung**

Die Erfindung betrifft eine temporär implantierbare Elektroden Anordnung, insbesondere eine Multi Elektroden Anordnung, die in einem Katheter integriert ist und die den linken Vorhof an unterschiedlichen Stellen abtastet (mapped) und stimuliert, um die Ausbreitung der physiologischen Signale bei Vorhofftimmem zu messen.

Die Elektroden Anordnung besteht aus einem geflochtenen Schirm aus Memory Metall Drähten. Mindestens ein Draht des Schirmes ist mit einer Wendel überzogen, wobei die Wendel aus mehreren parallel verlaufenden isolierten Drähten gefertigt ist, wobei mindestens ein Draht einen Pol trägt, der durch punktförmiges Entfernen der Isolationsschicht auf der Wendel gebildet wird.

## Beschreibung

### Die Erfindung:

Die Erfindung betrifft eine temporär implantierbare Elektroden Anordnung, insbesondere eine Multi Elektroden Anordnung, die den linken Vorhof an unterschiedlichen Stellen abtastet (mapped) und stimuliert, um die Ausbreitung der physiologischen Signale bei Vorhofflimmem zu messen.

### Probleme:

Vorhofflimmem tritt relativ häufig auf. Insgesamt scheint Vorhofflimmem in den letzten Jahren zugenommen zu haben, was mit einem immer älter werdenden Patientenkollektiv erklärt wird. Vorhofflimmem führt zu einer schnellen Überleitung der Erregung auf die Ventrikel, so dass es zu akuter hämodynamischer Instabilität kommen kann. Die Beseitigung von Vorhofflimmem durch Hochfrequenz Ablation erfolgt bisher durch einen im linken Vorhof eingeführten Katheter, der nach der sogenannten "Mace Prozedur" neben den Ausgängen der Pulmonal Venen auch andere Teile im linken Vorhof ablatiert. Wichtig dabei ist es, dass die Ablation nicht punktförmig, sondem in Linien erfolgt. Die genaue Ausbreitung und Ablation der elektrophysiologischen Signale im linken Vorhof ist noch nicht genau erforscht und von Patient zu Patient unterschiedlich. Um die Belastung des Patienten möglichst gering zu halten, bedarf es an mehr physiologischer Informationen im linken Vorhof. Unter WO 20051112813 A1 wird ein Katheter beschrieben, der ein aus Memory Metall geflochtenen Schirm enthält, dessen einzelne Drähte isoliert und durch Entfernen einzelner isolierter Stellen pro Flechtdraht ein Pol entsteht. Die Wärme Behandlung des aus einer Vielzahl von Nitinol Drähten bestehenden Schirms liegt bekanntlich bei etwa 500° C. Derartige Temperaturen sind für Materialien, die eine elektrische Isolation der einzelnen Drähte garantieren sollen, zur Zeit technisch nicht realisierbar. Wie eine nachträgliche Isolation der Drähte nach der Wärme Behandlung erfolgen soll, wurde nicht beschrieben.

### Lösungsweg:

Es besteht daher die Aufgabe, eine Elektrodenanordnung der eingangs beschriebenen Art zu schaffen, mit dessen Hilfe der linke Vorhof abgetastet wird, um damit das Verständnis über Herkunft, Ausbreitung und den Verlauf der elektrophysiologischen Signale zu verbessern. Zu diesem Zweck wird ein aus Memory Metall (Nitinol) geflochtener Schirm mit mindestens einem Elektroden Pol, vorzugsweise mit einem Areal von einer Vielzahl von Elektroden Polen ausgestattet und distal an einem Katheter befestigt, der transseptal in den linken Vorhof eingeführt wird. Zum Unterschied zu WO 20051112813 A1 bilden nicht die einzelnen Drähte des Schirmes die Pole, sondem nach der Wärme Behandlung werden über einzelne Drähte des Schirmes, Wendeln, die aus einer parallelen Anzahl von isolierten Drähten gefertigt sind aufgebracht und die Enden proximal an einem Handgriff herausgeführt. An den Stellen der Wendel, an der ein Pol gebildet werden soll, wird die Isolationsschicht punktförmig entfernt und das blanke Metall bildet dann den gewünschten Pol. Beispielsweise kann man mit einer aus acht Drähten gewickelten Wendel acht Pole erzeugen. Benutzt man nur 5 der etwa 20 bis 80 Schirmdrähte, so erhält man bereits 40 Pole und die darüber hinaus, durch Drehen des Schaftes den linken Vorhof genau abtasten. Das Schirm Geflecht wird hier nur als Träger und Formgeber benutzt. Es kann aber auch als indifferente Elektrode benutzt werden.

### Vorteile:

Der Vorteil der Erfindung liegt darin, dass mit einem Schirm Geflecht eine multipolare Elektrode herstellbar ist, die über einen Katheter transseptal in den linken Vorhof eingeführt werden kann und dort die Ausbreitung elektrophysiologischer Signale verfolgen kann.

### Abbildungen:

Weitere Einzelheiten, Merkmale und Vorteile der Erfindung lassen sich dem folgenden Beschreibungsteil entnehmen, in dem anhand der Zeichnungen und Ausführungsbeispiele die Erfindung näher erläutert wird. Sie zeigen in schematischer Darstellung in
Fig.1 eine Ansicht des linken Vorhof des Herzens mit einer platzierten Elektroden Anordnung in der Form eines flachen kreisförmigen Geflechtes.
Fig.2 eine detaillierte Vorderansicht einer beispielhaften Elektroden Anordnung mit fünf Pol Armen des Geflecht Schirmes.
Fig.3 eine detaillierte acht-fache (8-fache) Wendel mit den entsprechenden Polen.
Fig.4 eine beispielhafte Elektroden Anordnung am Eingang eines Blutgefäßes (z.B. Vene im linken Vorhof) (hier nicht vollständig dargestellt).

### Beschreibung der Abbildungen:

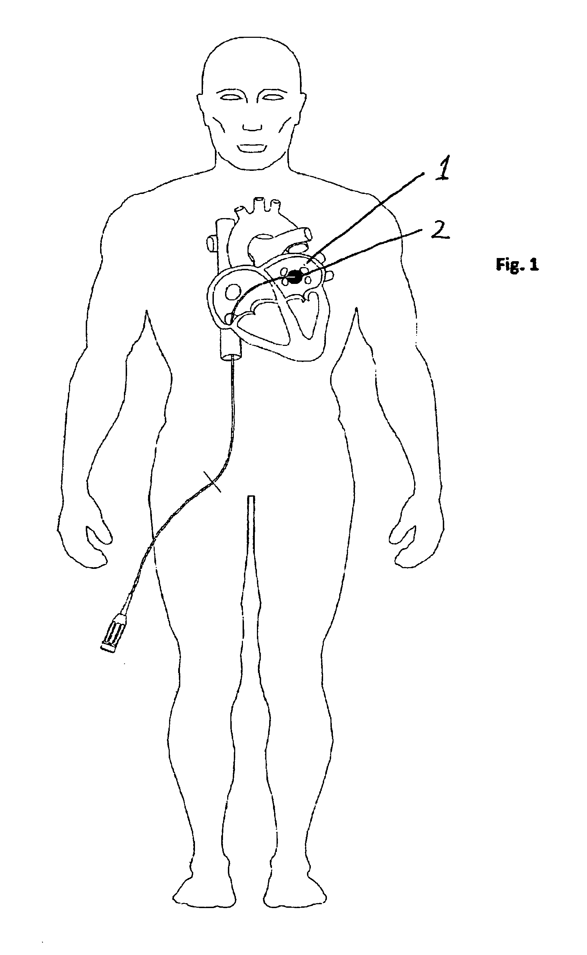

Fig.1 zeigt eine Ansicht des linken Vorhofs (1) des Herzens mit einer platzierten Elektroden Anordnung (2) In der Form eines flachen kreisförmigen Geflechts. Das Geflecht kann auch andere Formen annehmen, z.B. Ellipsen Form.

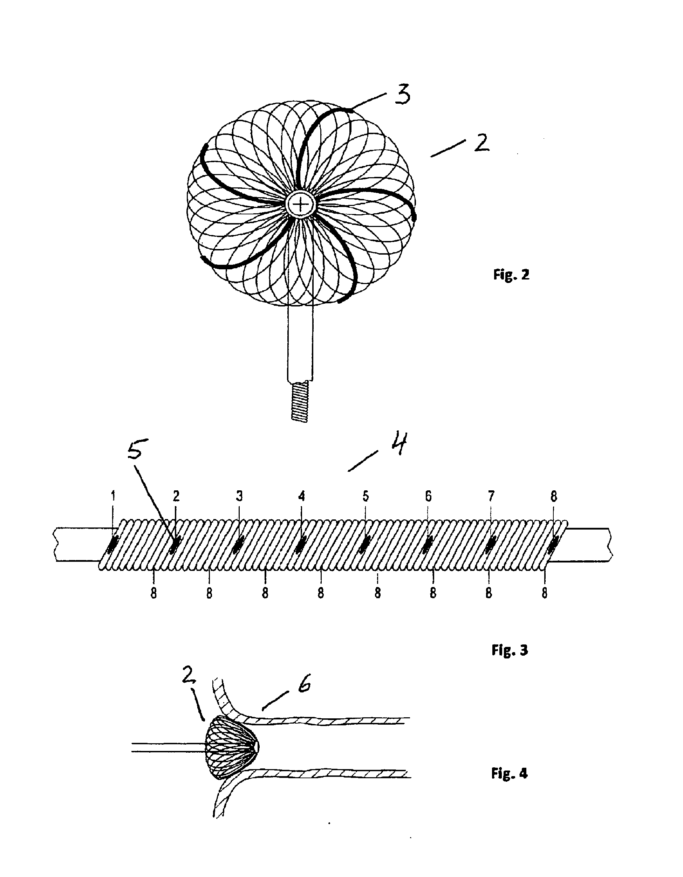

Fig.2 zeigt eine detaillierte Vorderansicht einer beispielhaften Elektroden Anordnung (2), bei der als Beispiel fünf Drähte des Geflecht Schirmes (3) selektiert wurden, die jeweils mit einer isolierten Wendel überzogen worden sind. Die Größe und Anzahl der Drähte des kreisförmigen Geflechts wird entsprechend den anatomischen Anforderungen selektiert. Die Drähte der Wendel sind beispielsweise aus Edelstahl, MP35N, Nitinol, Gold, Platin, Isotan oder Wolfram. Die Isolation der Drähte besteht beispielsweise aus Polyimid, Polyurethan, Pebax, PTFE, Polyamid oder anderen biokompatiblen Kunststoffen.

Fig.3 zeigt beispielhaft eine detaillierte Ansicht einer isolierten flexiblen acht-fach Wendel (4) mit acht Polen (5). Die Anzahl und Größe der Pole wurde durch selektives Entfernen der Isolierung der entsprechenden Wendeldrähte erzeugt. Die achtfache Wendel muss sich auf jedem der fünf Wendeldrähte sieben Mal wiederholen, damit die 8 voneinander isolierten Pole zustande kommen. Bei der Anordnung in Fig. 2 ergeben sich damit 40 Pole. Die Oberfläche der Pole können sowohl als blanker Draht oder durch Auftragen einer Schicht aus Edelmetall Pulver (z.B. Gold) mit Hilfe eines LASERs, genutzt werden.

Fig.4 zeigt eine Elektroden Anordnung (2), die in einem Eingang zu einem Blutgefäß (6) platziert ist (z.B. Pulmonal Vene im linken Vorhof). Durch Auswahl der Anzahl und Durchmesser der Nitinol Drähte, aus denen ein Schirm geflochten wird, kann die Elastizität bestimmt werden. Wird ein Geflecht Schirm beispielsweise aus 20 Nitinol Drähten mit einem Draht Durchmesser von 0,1 mm hergestellt und bestehen die Pol Wendeln aus isolierten Gold Drähten zwischen 0,050 und 0,1 mm, so wird sein Verhalten so elastisch, dass er leicht in ein Blutgefäß eingeführt werden kann.

## Patentansprüche

1. Temporär implantierbare Elektroden Anordnung, die in einem Katheter integriert ist und die den linken Vorhof an unterschiedlichen Stellen abtastet (mapped) und stimuliert, **dadurch gekennzeichnet,**
**dass** die Elektroden Anordnung aus einem geflochtenen Schirm aus Memory Metall Drähten besteht, wobei mindestens ein Draht des Schirmes mit einer Wendel überzogen ist, wobei die Wendel aus mehreren parallel verlaufenden isolierten Drähten gefertigt ist, wobei mindestens ein Draht einen Pol trägt, der durch punktförmiges Entfernen der Isolationsschicht auf der Wendel gebildet wird.

2. Elektroden Anordnung nach Anspruch 1, **dadurch gekennzeichnet,**
**dass** die Elektrodenanordnung multipolar aufgebaut ist und die Wendel mehr als einen Pol hat.

3. Elektroden Anordnung nach Anspruch 1 oder 2, **dadurch gekennzeichnet,**
**dass** mehr als ein Draht des Schirmes mit einer Wendel überzogen ist.

4. Elektroden Anordnung nach Anspruch 1 bis 3, **dadurch gekennzeichnet,**
**dass** die Enden der Wendeldrähte durch einen Katheter zu einem Katheter Handgriff geführt werden.

5. Elektroden Anordnung nach Anspruch 1 bis 4, **dadurch gekennzeichnet,**
**dass** der Pol blanker Draht ist oder blanker Wendeldraht auf den zusätzlich eine Schicht aus Edelmetallpulver aufgetragen wurde.

6. Elektroden Anordnung nach Anspruch 1 bis 5, **dadurch gekennzeichnet,**
**dass** die Drähte der Wendel aus Edelstahl, MP35N, Elgiloy, Gold, Silber, Platin, Isotan, Titan Wolfram oder deren Legierungen bestehen.

7. Elektroden Anordnung nach Anspruch 1 bis 6, **dadurch gekennzeichnet,**
**dass** die Isolationsschicht aus Polyimid, Silikon, PTFE, Pebax, Polyurethan, Parylene, Polyamid oder einem biokompatiblen Lack besteht.

8. Elektroden Anordnung nach Anspruch 1 bis 7, **dadurch gekennzeichnet,**
**dass** der geflochtene Schirm kreis- oder ellipsenförmig ist

9. Elektroden Anordnung nach Anspruch 1 bis 8, **dadurch gekennzeichnet,**
**dass** die Elastizität der Elektroden Anordnung derart ist, dass die Elektroden Anordnung in ein Blutgefäß eingeführt werden kann.
